# EUROPÄISCHE PATENTANMELDUNG

(11) **EP 1 043 392 A2**
(43) Veröffentlichungstag der Anmeldung: **11.10.2000**
(21) Anmeldenummer: 00103579.9
(22) Anmeldetag: 19.02.2000
(51) Int. Cl.: C12G 3/06, C11B 9/02

(54) **Digestif aus Wasser, Ethanol, ätherischen Ölen, Aromen und Bitterstoffen sowie Verfahren zu seiner Herstellung**

(30) Priorität: 07.04.1999 DE 19915560
(71) Anmelder: Popp, Michael, Prof. Dr., 92318 Neumarkt (DE)
(72) Erfinder: Popp, Michael, Prof. Dr., 92318 Neumarkt (DE)
(74) Vertreter: Matschkur, Peter

(57) **Zusammenfassung**

Verfahren zur Herstellung von Digestiva aus Wasser, Ethanol, ätherischen Ölen, Aromen und Bitterstoffen, wobei bei der Extraktion von Wirkstoffen aus Pflanzen für Arzneimittel, Kosmetika oder andere pharmazeutische Produkte anfallende ätherisch wässrige Lösungen auf die übliche Trinkstärke gebracht und gegebenenfalls mit Zusatzstoffen versetzt und/oder anderen Lösungen verschnitten werden.

## Beschreibung

Die Erfindung bezieht sich auf ein Digestif aus Wasser, Ethanol, ätherischen Ölen, Aromen und Bitterstoffen sowie ein Verfahren zu seiner Herstellung.

Derartige Digestiva mit einem hohen Anteil an ätherischen Ölen, Aromen und Bitterstoffen sind seit Jahrhunderten dafür bekannt, dass sie bei Einnahme vor bzw. nach einer Mahlzeit die Sekretion von Verdauungssäften steigern und somit insbesondere schwer verdauliche Speisen bekömmlicher machen.

Die klassischen Verfahren zur Herstellung von Digestiva gehen entweder aus von einer Beimischung der entsprechenden ätherischen Öle, Aromen oder Bitterstoffe zu einem Ethanol-Wasser-Gemisch, einem einfachen Ansatz, bei dem entsprechend Ausgangspflanzen od. dgl. dem Ethanol-Wasser-Gemisch beigefügt werden und nach einiger Zeit, in der die gewünschten Stoffe übergangen sind, wieder abgetrennt werden und schließlich von einer Destillation von ätherisch ölhaltigen Produkten und der anschließenden Aufbereitung von trinkfähigen Präparaten. Beim Destillationsverfahren erfolgt die Destillation aus dem Ansatz der zu extrahierenden Pflanze mit dem Ethanol-Wasser-Gemisch. Dieses Verfahren ist sehr aufwendig und bei der ausschließlichen Extraktion der Inhaltsstoffe zur Digestifbereitung sehr teuer.

Der Erfindung liegt daher die Aufgabe zugrunde, ein Digestif und ein zu seiner Herstellung geeignetes Verfahren zu schaffen, das eine bessere Ausnutzung der Rohstoffe ermöglicht und geringe Herstellkosten verursacht.

Zur Lösung dieser Aufgabe ist erfindungsgemäß vorgesehen, dass er als Basis einer auf übliche Trinkstärke gebrachtes Ethanol-Wasser-Gemisch enthält, das bei der Extraktion von Wirkstoffen aus Pflanzen für Arzneimittel, Kosmetika und andere pharmazeutische Produkte anfällt. Zur Herstellung ist erfindungsgemäß vorgesehen, das bei der Extraktion von Wirkstoffen aus Pflanzen für Arzneimittel, Kosmetika oder andere pharmazeutische Produkte anfallende ätherisch wässrige Lösungen auf die übliche Trinkstärke gebracht und gegebenenfalls mit Zusatzstoffen versetzt und/oder anderen Lösungen verschnitten werden.

Die Erfindung geht dabei von der Erkenntnis aus, dass bisher bei der Herstellung von Extrakten, sei es aus Frischpflanzen oder aus getrockneten Drogen, stets Ethanol-Wasser-Lösungen anfallen, die dabei - zur besseren Ausnutzung - entweder nochmals zur Tinkturherstellung benutzt werden oder zur Wiederaufbereitung an Destillationseinrichtungen der Bundesmonopolverwaltung gegeben wurden. Auch bei der Wiederverwendung für einen zweiten Ansatz landet die Ethanol-Wasser-Mischung mit den beigemischten ätherischen Ölen zur Aufbereitung in den Destillationseinrichtungen der Bundesmonopolverwaltung, was zu einer erheblichen Kostenbelastung führt.

Die Erfindung geht nunmehr von der weiteren Erkenntnis aus, dass diese bei der Wirkstoffherstellung aus Pflanzen anfallenden Destillate noch einen erheblichen Anteil an ätherischen Ölen, Aromen und sonstigen Inhaltsstoffen der Pflanzen enthalten, die das Destillat unmittelbar - d. h. allenfalls nach einer vorherigen Verbringung auf eine übliche Trinkstärke - als Digestif verwendbar machen. Man muss das Destillat also nicht aufarbeiten, um die Inhaltsstoffe zu entfernen, um aus dem Destillat lediglich billigen Industriealkohol zu machen, sondern man kann das Destillat direkt als Ausgangsbasis für die Herstellung eines Digestifs verwenden. Da es sich bei der eingesetzten Ausgangsproduktion zur Herstellung von Wirkstoffen, beispielsweise für die Arzneimittelindustrie um Fluidextrakte bzw. Tinkturen handeln, die nach den gängigen Extraktionsverfahren hergestellt werden und deren Extraktionsmittel ja pharmazeutische Ethylalkohol-Wasser-Mischungen sind, kann davon ausgegangen werden, dass schädliche Beiprodukte in den Destillation nicht nachgewiesen werden können. Demzufolge eignen sich diese Destillate unmittelbar zum menschlichen Verzehr und können durch geringfügige Aufarbeitung (Aufkonzentration oder Verdünnung) sowie gegebenenfalls die Beimischung oder den Verschnitt mit anderen Stoffen unmittelbar zu einem Digestif verarbeitet werden.

Durch diese Herstellung eines Digestifs unmittelbar im Zusammenhang mit der Extraktion von Pflanzen zur Herstellung von Wirkstoffen für Arzneimittel, Kosmetika und andere pharmazeutische Produkte ergibt sich ein wesentlich kostengünstigeres Verfahren als die bisherigen Separat-Destillationen, wobei ja noch hinzukommt, dass das Ausgangsdestillat für den Digestif ja an sich ein Abfallprodukt eines anderen Prozesses ist, das bisher ja noch gesondert unter Aufwendung weiterer Kosten aufbereitet werden mußte, um wenigstens (billigen) Industriealkohol herzustellen. Darüber hinaus ergeben sich besonders reine, also von unerwünschten Beimengungen frei und inhaltsreiche Digestiva, da bei der Extraktion von Wirkstoffen von gereinigten durch Absetzen und Abfiltrieren von allen Feststoffbeimischungen befreite Tinkturen verwendet werden, und nicht wie bei der bisherigen Digestif-Herstellung direkt aus der Pflanzenbrühe destilliert wird.

In Weiterbildung der Erfindung werden die zu extrahierenden Pflanzen, und zwar unabhängig davon, ob sie als Frischpflanzen oder als getrocknete Drogen verarbeitet werden, mit einem Ethanol-Wasser-Gemisch angesetzt, die ausgelaugten Pflanzenreste abgetrennt und aus der gewonnenen Tinktur durch Verdampfen und Trocknen der native Extrakt der Wirkstoffe gewonnen, wobei das anfallende Destillat, bzw. Redestillat bei mehrfacher Destillation, unmittelbar als Basis für einen Digestif verwendet wird.

Um einen hohen Anteil an ätherischen Ölen im Produkt zu erhalten, kann dabei in weiterer Ausgestaltung der Erfindung vorgesehen sein, dass bei der Destillation der Tinktur zur Gewinnung der Wirkstoffe die Vor- und Rücklauftemperatur zwischen 90°C und 70°C und die Innentemperatur der Destillationsblase zwischen 25°C und 70°C und die Abluftfiltertemperatur zwischen 20°C und 40°C bei einem Druck zwischen 30 und 70 mbar gehalten wird. Bei dieser Arbeitsweise ergibt sich ein Destillat, das einen hohen Anteil an ätherischen Ölen, Aromen und gegebenenfalls Bitterstoffen aufweist, so dass es sich ohne weitere besondere Aufbereitung für einen hochwertigen Digestif verwenden lässt. Man bringt lediglich das anfallende Destillat nach einer gegebenenfalls zusätzlichen Filtration, auf Alkoholgehalte zwischen 20 % und 90 % Alkohol, vorzugsweise zwischen 35 % und 70 % Alkohol und insbesondere zwischen 40 % und 60 % Alkohol. Dabei liegt es im Rahmen der Erfindung, mehrere ethanolisch ölhaltige Drogen als Ausgangsprodukte einzusetzen, wie beispielsweise Anis, Fenchel, Kümmel, Wacholder, Kamille, Eukalyptus, Apfel, Birne und den entsprechenden Tresterarten sowie Echinacea, Agnus castus, Allium cepa und Allium sativum. Der Digestif kann dabei als Mischgetränk aus den verschiedenen Einzeldestillaten komponiert werden.

Grundsätzlich eignet sich das erfindungsgemäße Verfahren für alle bei der Herstellung von Wirkstoffen aus Pflanzen eingesetzte Destillationsverfahren, also beispielsweise Dünnschichtverfahren, Vakuum-Aufkonzentrations-Verfahren, Fallstromverfahren, Vakuumbandtrocknung oder alle weiteren Verfahren, die zur Konzentration verwendet werden und ein Auffanggefäß für das Ethanol-Wasser-Gemisch besitzen.

### Beispiel:

Zur Herstellung von Trockenextrakt, z. B. Herba Thymi (Thymiankraut) wir 70 %-iger Ethanol mit den Frischpflanzen oder der getrockneten Droge aus den Pflanzen vermischt und zur Herstellung einer entsprechenden Ausgangstinktur verwendet. Hierzu wird nach einer ausreichenden Einwirkzeit, bei der gegebenenfalls eine zusätzliche Erwärmung stattfinden kann, eine Filtration vorgenommen, mit der die Pflanzenteile abgetrennt werden. Gegebenenfalls nach einer weiteren Absetzzeit und Filtration der ausgefallenen feinen Festkörperschwebteilchen wird die so gewonnene reine Tinktur einem Trocknungs- oder Destillationsverfahren unterworfen, bei welchem die gewünschten Wirkstoffe der Ausgangspflanze, also im angesprochenen Beispiel des Thymiankrautes, als nativer Extrakt gewonnen werden. Das dabei anfallende Destillat, das bisher ein Abfallprodukt bildete, das gesondert wieder aufbereitet werden musste, wird erfindungsgemäß unmittelbar als Digestif verwendet, wobei lediglich eine Verbringung auf einen üblichen Trinkalkoholgehalt erforderlich ist. Selbstverständlich kann man einen solchen Digestif mit anderen in gleicher Weise gewonnenen Digestiva verschneiden oder auch auf Wunsch gesonderte Geschmacksstoffe noch zufügen. Der besondere wirtschaftliche Vorteil des erfindungsgemäßen Verfahrens zur Herstellung von Digestiva liegt darin, dass zeitnah und parallel in einem Ablauf zwei unterschiedliche Produkte hergestellt werden können, nämlich einmal beispielsweise ein Arzneimittelwirkstoff und aus den bisher als Abfallstoff betrachteten Destillaten ein Genussmittel. Die aufwendige bisherige Aufbereitung des Ethanols entfällt.

## Patentansprüche

1. Verfahren zur Herstellung von Digestiva aus Wasser, Ethanol, ätherischen Ölen, Aromen und Bitterstoffen, dadurch gekennzeichnet, dass bei der Extraktion von Wirkstoffen aus Pflanzen für Arzneimittel, Kosmetika oder andere pharmazeutische Produkte anfallende ätherisch wässrige Lösungen auf die übliche Trinkstärke gebracht und gegebenenfalls mit Zusatzstoffen versetzt und/oder anderen Lösungen verschnitten werden.

2. Verfahren nach Anspruch 1, dadurch gekennzeichnet, dass die zu extrahierenden Pflanzen mit einem Ethanol-Wasser-Gemisch angesetzt werden, dass die ausgelaugten Pflanzenreste abgetrennt und aus der gewonnenen Tinktur durch Verdampfen und Trocknen der native Extrakt der Wirkstoffe gewonnen wird, wobei das anfallende Destillat, bzw. Redestillat bei mehrfacher Destillation, unmittelbar als Basis für einen Digestif verwendet wird.

3. Verfahren nach Anspruch 1 oder 2, dadurch gekennzeichnet, dass bei der Destillation der Tinktur zur Gewinnung der Wirkstoffe, die Vor- und Rücklauftemperatur zwischen 90°C und 70°C, die Innentemperatur der Destillationsblase zwischen 25°C und 70°C und die Abluftfiltertemperatur zwischen 20°C und 40°C bei einem Druck zwischen 30 und 70 mbar gehalten wird.

4. Verfahren nach einem der Ansprüche 1 bis 3, dadurch gekennzeichnet, dass man das anfallende Destillat nach der Filtration auf Alkoholgehalte zwischen 20 % und 90 % Alkohol, vorzugsweise zwischen 35 % und 70 % Alkohol und insbesondere zwischen 40 % und 60 % Alkohol einstellt.

5. Verfahren nach einem der Ansprüche 1 bis 4, dadurch gekennzeichnet, dass man mehrere ethanolisch ölhaltige Drogen als Ausgangsprodukte einsetzt, wie z. B. Anis, Fenchel, Kümmel, Wacholder, Kamille, Eukalyptus, Apfel, Birne und den entsprechenden Tresterarten, sowie Echinacea, Agnus castus, Allium cepa und Allium sativum.

6. Digestif aus Wasser, Ethanol, ätherischen Ölen, Aromen und Bitterstoffen, dadurch gekennzeichnet, dass er als Basis einer auf übliche Trinkstärke gebrachtes Ethanol-Wasser-Gemisch enthält, das bei der Extraktion von Wirkstoffen aus Pflanzen für Arzneimittel, Kosmetika und andere pharmazeutische Produkte anfällt.

7. Verwendung eines Ethanol-Wasser-Gemisch, das bei der Extraktion von Wirkstoffen aus Pflanzen für Arzneimittel, Kosmetika und andere pharmazeutische Produkte anfällt als Basis für einen Digestif.
